# EUROPEAN PATENT APPLICATION

(11) **EP 4 470 390 A1**
(43) Date of publication of application: **04.12.2024**
(21) Application number: 23382514.0
(22) Date of filing: 31.05.2023
(51) Int. Cl.: A23L 3/00, A23L 3/10, A61L 2/07

(54) **CIRCULAR RESOURCES SYSTEM AND METHOD**

(71) Applicant: Urtech Water S.L., 31013 Pamplona (ES); Ferlo Soluciones de Proceso, S.L., 31570 San Adrián, Navarra (ES)
(72) Inventor: López Palacios, Daniel, PAMPLONA, NAVARRA (ES); López Domínguez, Rafael, SAN ADRIÁN, NAVARRA (ES); López Palacios, Javier, PAMPLONA, NAVARRA (ES)
(74) Representative: Elion IP, S.L.

(57) **Abstract**

A retort system (100) comprising: at least one autoclave (10) for sterilizing a product contained within a cavity of the at least one autoclave (10), wherein in use of the retort system (100), the cavity of the at least one autoclave (10) is partially filled with water, the product being sterilized by transferring heat from said water; a first heat exchanger (20) configured to cool down the water remaining within the cavity of the at least one autoclave (10) after a sterilization cycle has been completed, the first heat exchanger (20) cooling down said water by transferring heat of said water to cooling water; a recovery system (25) comprising: a first tank (40) configured to store water coming from the cavity of the at least one autoclave (10) after a sterilization cycle has been completed and the remaining water within the cavity has been cooled down; a second heat exchanger (30) configured to transfer the heat previously transferred to the cooling water at the first heat exchanger (20) to the water stored in the first tank (40); and a second tank (50) configured to store the water heated at the second heat exchanger.

## Description

### TECHNICAL FIELD

The present invention relates to systems and methods for sterilizing products, such as foodstaff (i.e. tinned food) or medical devices. More specifically, it relates to systems and methods for saving water and/or energy in installations comprising at least one system for sterilizing products, such as at least one autoclave.

### STATE OF THE ART

Retort sterilization is a process in which an object is sealed in a package and the package is in turn disposed within a container (also referred to as autoclave). The inside of the container is then submitted to heat (for example, between 108 and 130 °C) and pressure (for example, a maximum pressure of about 2.4 bar) for several minutes. To achieve the desired sterilization in the object, suitable conditions must be applied, which may depend on the type of object, so that the desired sterilization is achieved while the quality of the object does not deteriorate along time and the package bears the applied temperature and pressure conditions and inner humidity.

Conventional retort systems are composed of one or more autoclaves, within which the products to be sterilize are disposed. Usually, retort systems are composed of a plurality of batch autoclaves. An autoclave is a container whose cavity can be hermetically sealed once packages containing the products to be sterilized are disposed within the container. Together with the packages, the container has water inside, because sterilization is typically achieved by applying heat and pressure within the cavity. Heat is normally transferred to the packages through water (liquid and/or vapour). In some autoclaves steam is also injected within the cavity and/or water is sprayed within the cavity to boost the mix of steam and water, so that homogeneous temperature distribution is achieved. This results in a uniform process throughout the autoclave. When a new sterilization cycle starts, water (typically osmosis water or at least softened water) is first added to the cavity of the container. This water is called overfilling water. The overfilling water can be heated in two different ways: by directly applying steam and/or through a heat exchanger associated to the autoclave. Once the desired temperature and pressure are reached (the pressure should reach about 2.4 bar to correctly sterilize the products), these temperature and pressure conditions are maintained for several minutes. This time depends on several parameters, such as the type of product to be sterilized, on the type of package, its size, size of autoclave, among others.

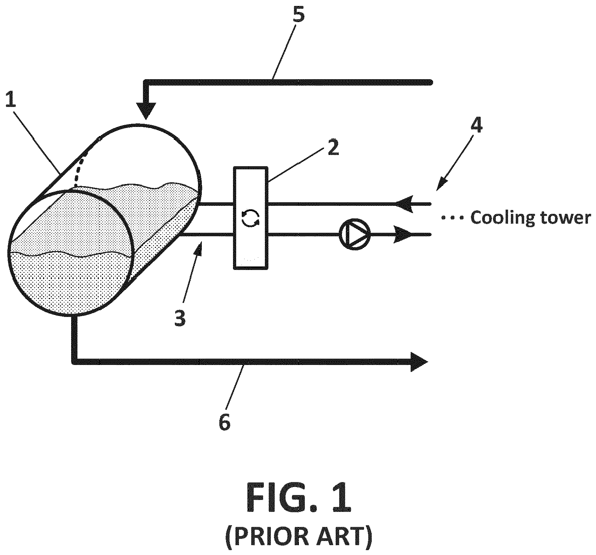

When a sterilization cycle is finished in an autoclave, the temperature and pressure reached within the cavity, which are typically of around 120-130°C and 2.4 bar, respectively, need to be reduced to safe levels prior to opening the autoclave and taking the packages containing the product out of the container of the autoclave. When the autoclave is opened, the overfilling water leaves the container at high temperature (but within a safe level) and is thrown away. The overfilling water within the cavity of the autoclave needs to be cooled down to a safe temperature level to empty the cavity, so that a new sterilization cycle can start as soon as possible. For this reason, conventional retort systems usually have a heat exchanger to cool down the water within the autoclave using water at ambient temperature, for example from a cooling tower. This is schematically illustrated in Figure 1, wherein an autoclave 1 containing hot water resulting from a sterilization cycle is shown. To favor the cooling down of the water within the container of the autoclave 1, it is circulated through the heat exchanger 2 along pipe circuitry 3 connecting the cavity of the autoclave 1 and the heat exchanger 2. Pipe circuitry 3 is composed of a pipe taking hot water from the autoclave 1 to the heat exchanger 2 (usually with the help of a pump, not illustrated) and another pipe returning cooled-down water from the heat exchanger 2 to the autoclave 1. The temperature of the hot water leaving the autoclave 1 varies, during the cooling-down process, from about 120°C to about 40°C. Simultaneously, cold water (i.e. ambient water, i.e. 17°C) is circulated through the heat exchanger 2 along pipe circuitry 4. Pipe circuitry 4 has a pipe taking cold water from, for example, a cooling tower, to the heat exchanger 2 and another pipe returning warmed water from the heat exchanger 2 to the, for example, cooling tower. The two pipe circuitries 3, 4 do not share water. This way, the water contained in the autoclave 1 is cooled down while its heat is transferred to the water circulating through the other pipe circuitry 4. When the temperature of the water contained in the autoclave 1 is sufficiently low (such us below 45°C, preferably between 20 and 38°C), the autoclave 1 can be emptied. An inlet pipe 5 to fill in the cavity of the autoclave 1 with suitable water every new cycle is shown. Suitable water is water that has been adequately treated. For example, osmosis water. An outlet pipe 6 to empty the cavity of the autoclave 1 when a sterilization cycle is finished is also shown.

Conventional autoclaves, like the one shown in Figure 1, have some drawbacks. The process of cooling down the water within the autoclave is relatively slow. Besides, a large amount of energy is wasted in the cooling down process, since the energy (heat) transferred to the water circulating in the second pipe circuitry 4 is wasted. Moreover, water is also misused, because water filling the autoclave 1 is thrown away after cooling down every new cycle is finished and new suitable water is used to refill the cavity prior to starting a new sterilization cycle.

These and other shortcomings tend to impair the effectiveness of current autoclave systems. Therefore, there is a need for a new system and method for energy and water recovery in installations comprising one or more autoclaves, that overcome the disadvantages of conventional autoclave systems.

### DESCRIPTION OF THE INVENTION

The present invention aims at overcoming the drawbacks of the prior art. The present invention provides a recovery system and method for recovering water and/or energy to be used in an installation or retort system comprising one or more autoclaves. The energy and water used in a sterilization process is thus reused in subsequent sterilization processes. The proposed recovery system and method reduce energy and water consumption by using a warm water tank that stores water to be subsequently reused and a hot water tank that stores energy in the form of hot water (energy which can be transferred to the one or more autoclaves).

Once sterilization has been completed, the product must be cooled to a condition close to the condition at the start of the sterilization process to allow safe opening of the autoclave. For this purpose, cooling water is used which, through a heat exchanger of each autoclave, is used to lower the internal temperature within the autoclave.

The sterilization process requires a large amount of energy to achieve the right working conditions. The proposed system and method enable the reuse of this energy for a next cycle of the autoclave or of another autoclave, reusing the energy available inside the autoclave to heat part of the process water and accumulate this water for the following cycles (energy that in conventional systems is wasted by cooling only the product of that cycle).

Energy is extracted during the cooling phase of the water kept in the cavity of an autoclave after a sterilization cycle. This energy is utilized for heating the water to be supplied to the cavity of the autoclave prior to starting a subsequent sterilization cycle. Heat is collected and stored in a hot water tank.

Regarding water, most of the water used in a sterilization process is also wasted at around 40°C, while new fresh water at about 17°C (and suitably treated) needs to be used in subsequent sterilization cycles. The proposed system and method enable also the reuse of water already used in previous sterilization cycles.

A control system manages and monitors the performance of the water and/or energy recovery system.

Retorts are at the heart of many shelf-stable food processing lines, such as: ready-meals (including meat meals, fish meals and others); baby food, pet food, soups and sauces; mushrooms, vegetables, beans and other fresh food; milk (plain, chocolate, milkshakes, cream, etc.); other beverages (such as fruit juices, non-carbonated beverages, tea, coffee, etc.); rigid containers (bottles, cans, etc.); semi-rigid containers (tubs, etc.) and flexible containers (pouches, etc.).

The one or more autoclaves of the retort system preferably sterilize edible products contained in containers, such as cans, bottles, tubs, pouches, or others. The autoclaves can sterilize other products different from foodstuff.

In a first aspect of the invention, a retort system is provided. The retort system comprises: at least one autoclave for sterilizing a product contained within a cavity of the at least one autoclave, wherein in use of the retort system, the cavity of the at least one autoclave is partially filled with water, the product being sterilized by transferring heat from said water under certain pressure conditions; a first heat exchanger configured to cool down the water remaining within the cavity of the at least one autoclave after a sterilization cycle has been completed, the first heat exchanger cooling down said water by transferring heat of said water to cooling water; and a recovery system. The recovery system comprises: a first tank configured to store water coming from the cavity of the at least one autoclave after a sterilization cycle has been completed and the remaining water within the cavity has been cooled down; a second heat exchanger configured to transfer the heat previously transferred to the cooling water at the first heat exchanger to the water stored in the first tank; and a second tank configured to store the water heated at the second heat exchanger.

In embodiments of the invention, the retort system further comprises: a first piping system disposed between the at least one autoclave and the first heat exchanger, the first piping system for taking water from the at least one autoclave to the heat exchanger and for returning cooled-down water from the heat exchanger to the autoclave; a second piping system disposed between the first heat exchanger and the second heat exchanger, the second piping system for circulating cooling water between the first heat exchanger and the second heat exchanger; and a third piping system for circulating the water stored in the first tank through the second heat exchanger so that said water is heated with the heat transferred from the water circulating through the second piping system.

In embodiments of the invention, the first tank is further configured to receive and store treated water different from the water coming from the cavity of the at least one autoclave.

In embodiments of the invention, the retort system further comprises a pipe for taking water from the second tank to the at least one autoclave prior to starting a sterilization cycle.

In embodiments of the invention, the retort system further comprises means for pumping water stored in the first water tank to the second water tank.

In embodiments of the invention, the retort system further comprises at least one temperature sensing means to measure the temperature at at least one point in the retort system.

In embodiments of the invention, the retort system further comprises a system for treating the water stored in the first water tank.

In embodiments of the invention, the cooling water comes from a cooling tower. A cooling tower is a tank in which air and water are brought into direct contact with each other in order to reduce the water's temperature. As this occurs, a volume of water is evaporated, reducing the temperature of the water being circulated through the tower.

In embodiments of the invention, the cooling water can be reused water, for example water previously used in the installation, including water used in other processes and water coming from a water treatment plant. This has the advantage of increasing even more the efficiency of the system in terms of water reuse. Having reused water in the cooling system implies applying a suitable water quality control (i.e. according to sanitary standards, such as Spanish RD 1620/2007 and European Rules 741/2020) to prevent problems of contamination, fouling or corrosion that may impair the efficiency of the process and/or damage the first heat exchanger. To achieve this, a water treatment and control system is used, so that the quality and balance of the reused water is monitored continuously and in real-time. The use of biocide chemicals is thus prevented.

In embodiments of the invention, the product to be sterilized is kept within one or more containers disposed within said cavity.

In embodiments of the invention, the product to be sterilized is an edible product.

In embodiments of the invention, the at least one autoclave is configured to sterilize a product by applying a temperature a pressure treatment for a certain time.

In a second aspect of the invention, a method for water and/or energy recovery in an installation comprising at least one autoclave for sterilizing a product contained with a cavity of the at least one autoclave, is provided. The method comprises:
disposing a product to be sterilized within the cavity of the at least one autoclave;
partially filling the cavity of the at least one autoclave with water;
sterilizing the product by applying a temperature and pressure treatment for a certain time;
after the product is sterilized, at a first heat exchanger cooling down the water comprised in the cavity of the at least one autoclave using cooling water while heat is transferred from the water comprised in the cavity to the cooling water;
at a second heat exchanger, using the heated cooling water to warm up water to be provided to a cavity of at least one autoclave prior to starting a subsequent sterilization cycle.

In embodiments of the invention, the method further comprises storing in a first tank water coming from the cavity of the at least one autoclave after a sterilization cycle has been completed and the remaining water within the cavity has been cooled down.

In embodiments of the invention, the method further comprises storing in a second tank the water warmed up at the second heat exchanger.

Among the advantages of the proposed system and method, regarding energy storage: no superheated water is sent back to the cooling tower, which reduces the energy consumption of the cooling tower, such as its fan. Cold water from the cooling tower may not be used during the first part of the cooling phase, thus reducing cooling tower load. The system collects energy from the hot process water during the first portion of the cooling phase. Regarding energy recovery, heat energy is transferred to the cold process water during the first portion of the come-up phase.

In sum, the proposed method and system optimize the productive cycle of autoclaves by reusing part of the residual energy existing at the end of each sterilization cycle and part of the water used in the sterilization process. Energy, water and economic savings are thus achieved.

The proposed system and method also enable real-time computation of the cost of the productive cycle of autoclaves, as well as digitalization and traceability of the cycle.

Additional advantages and features of the invention will become apparent from the detail description that follows and will be particularly pointed out in the appended claims.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 schematically illustrates a conventional retort system comprising an autoclave and a heat exchanger.
Figure 2 shows a retort system including a water and energy recovery system according to embodiments of the present invention.

### DESCRIPTION OF A WAY OF CARRYING OUT THE INVENTION

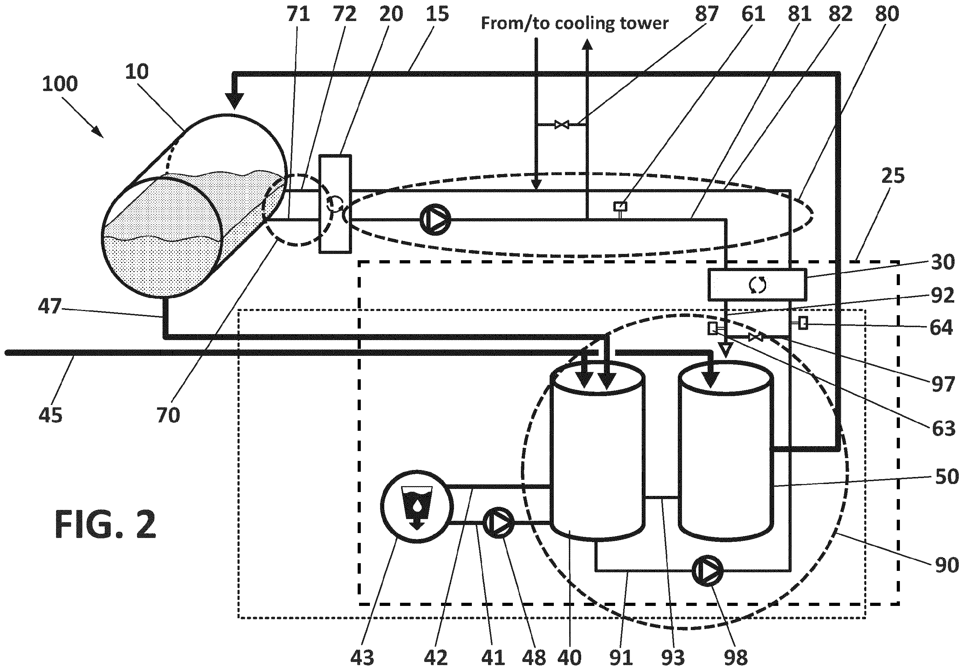

A recovery system 25 for recovering water and/or energy according to the present invention, to be used in a retort system or installation 100 having one or more autoclaves 10, is schematically shown in Figure 2. The retort system 100 is a fully automated retort that processes containers kept in its cavity, such as cans of any size and volume, from large foodservice cans to small retail cans. In the figure, a single autoclave 10 is shown. However, the retort system 100 may have a plurality of batch autoclaves. The autoclave 10 sterilizes products, such as foods, contained in the containers. Non-limiting examples of food being sterilized are beans, fruits, vegetables, meat, soups and pet food, among others.

The autoclave 10 is a conventional one in which products are sterilized by applying a temperature and pressure treatment for a certain time. The products (for example cans containing food) are disposed within the cavity of the autoclave 10 and the cavity is filled with suitable water up to a certain level (also referred to as overfilling water). Suitable water is water that has been adequately treated, i.e. osmosis water or softened water. The products can be disposed in packages comprising a certain number of containers (i.e. cans). The cavity is then hermetically sealed and the inside of the autoclave is then submitted to heat, typically between 108 and 130 °C, and pressure (around 2 bars of relative pressure, reaching a maximum pressure of about 2.4 bars) usually for several minutes. Heat is transferred to the products to be sterilized through water (liquid and/or vapour). Every time a new sterilization cycle starts, the autoclave 10 receives an amount of water through an inlet connected to an inlet pipe 15. The recovery system 25 permits to recover the overfilling water used in previous cycles and to reuse it through inlet pipe 15, as will be explained later, unlike in prior art systems, in which overfilling water is wasted. Depending on the type and design of the autoclave, an amount of water varying between 500 and 2000 liters of overfilling water can be recovered per autoclave and cycle. Furthermore, this overfilling water is treated water, such as osmosis water or softened water. Therefore, recovering this water is doubly important. The recovery process carried out at the recovery system 25 is explained next. After a sterilization cycle has ended, and once the temperature of the hot water (at about 108-130°C) remaining within the cavity of the autoclave 10 has been reduced (for example, to a temperature below 45°C, such as between 20 and 38°C), the cavity is emptied (in particular, the overfilling water is taken out of the cavity) through an outlet connected to an outlet pipe 47 and the sterilized containers or packages of containers can be taken out of the autoclave 10. However, the overfilling water taken out of the autoclave 10 is not thrown away, but conducted by outlet pipe 47 to a tank 40, 50, as is explained later, so that water is not wasted and can be recovered.

The cooling process for reducing the temperature of the hot water remaining within the cavity of the autoclave 10 once a sterilization cycle has ended is explained next. The retort system 100 has a heat exchanger 20 used to cool down the water within the autoclave 10 using cold water (for example, water at ambient temperature, such as water at about 17°C) obtained for example from a cooling tower (not shown). Hot water retained within the cavity of the autoclave 10 is circulated through the heat exchanger 20 along pipe circuitry 70 connecting the cavity of the autoclave 10 and the heat exchanger 20. Pipe circuitry 70 is composed of a pipe 71 taking hot water from the autoclave 10 to the heat exchanger 20 and another pipe 72 returning cooled-down water from the heat exchanger 20 to the autoclave 10. For example, the temperature of the water circulating through pipe circuitry 70 varies between 121 and 45°C. Simultaneously, cold water, for example stored in a cooling tower (not shown), is circulated through the heat exchanger 20 along pipe circuitry 80. Water travelling within circuitry 70 (from the autoclave 10 to the heat exchanger 20 and from the heat exchanger 20 to the autoclave 10) is not allowed to mix with cooling water circulating within pipe circuitry 80 because cooling water has not been treated to make it suitable (i.e. osmosis water or softened water) for the sterilization process being carried out in the autoclave 10. Pipe circuitry 80 has a pipe 82 taking cold water from the cooling tower to the heat exchanger 20 and another pipe 81 returning warmed-up water from the heat exchanger 20 to the cooling tower. More specifically, water in pipe circuitry 80 travels between the heat exchanger 20 and another heat exchanger 30. As explained, the two pipe circuitries 70, 80 do not share or mix or exchange water. This way, hot water contained in the autoclave 10 is cooled down at the heat exchanger 20 while its heat is transferred to water circulating through the other pipe circuitry 80. In this process, water circulating in the pipe circuitry 80 is in turn warmed up until it reaches a maximum temperature of 121°C. Usually, the temperature circulating through circuitry 80 reaches a value of, for example, between 70 and 95°, such as between 75 and 90°C, for example about 85°C. A bypass pipe 87 is disposed between pipes 81 and 82 in pipe circuitry 80 to be used in the event the cooling tower should be bypassed. This may happen, for example, at the beginning of the cooling cycle, such as during a period of time, dependent on the application, that may last between 5 and 10 minutes, when the heat exchanger 30 is recovering energy and therefore the bypass is closed (water does not circulate through the cooling tower). After this period of time, water must be circulated through the cooling tower so that water in the autoclave 10 is cooled down (bypass open).

The retort system 100 may be equipped with a controller (not shown) which manages the operation of the autoclaves and the recovery system. The controller may be implemented by a processor, a plurality of processors, a PLC, or the like. The retort system 100 may include an automatic loading and unloading system (not shown), also controlled by the process controller, which provides the customer with food safety, basket tracking, equipment reliability and labor savings.

Figure 2 also shows schematically several pumping means (i.e. pumps) to propel water from one point to another one in the installation. The installation may include other pumps (not shown). The installation may also include conventional valves at different points of the installation, to enable/disable the travelling of water, depending on the cycle being carried out.

The retort system 100, and in particular the recovery system 25, is also comprised of two water tanks 40, 50. One of the water tanks 40 is a lower temperature water tank, also referred to as warm water tank, meaning that it stores water at a maximum temperature of about 45°C. The main goal of this tank 40 is to save water by storing overfilling water from the cavity of the autoclave 10 which has already been cooled-down and which otherwise would be thrown away every time a sterilization cycle is completed. The other water tank 50 is a higher temperature water tank, also referred to as hot water tank, meaning that it stores water which temperature may vary between 50 and 90°C, such as between 50 and 75°C, as will be explained later. The main goal of this tank 50 is to recover some energy (in the form of hot water) obtained in the second heat exchanger 30 from the water circulating within pipe circuitry 80, which in turn has received this heat at the first heat exchanger 20. The terms "lower" and "higher" in respective lower temperature water tank 40 and higher temperature water tank 50 should be understood as relative to each other, meaning that the temperature of the water stored in the first tank 40 is lower (preferably at a maximum temperature of about 45°C) than the temperature of the water stored in the second tank 50 (preferably between 50 and 90 °C). In particular, during an water and/or energy recovery cycle (also referred as cooling cycle regarding the overfilling water within the cavity of the autoclave 10), after a sterilization cycle, the temperature of the water stored in the second tank 50 is normally above 60°C, because water circulating through the recovery system 25 is heated at heat exchanger 30 using the heat of the water circulating through pipe circuitry 80. The temperature of the water stored in the second tank 50 usually decreases, for example to temperature levels close to 50°C or even lower ones, when the installation is not in operation (for example at night or during the weekend) and therefore water in tank 50 gets colder.

The first time the autoclave 10 is used, both tanks 40, 50 are empty. In this case, the warm water tank 40 needs to be filled with suitable water (processed water, such as osmosis water or softened water) through pipe 45. The temperature of this water may vary between 10-20°C, such as between 12-17°C. After a first sterilization cycle and in subsequent sterilization cycles in the autoclave 10, the warm water tank 40 receives cooled-down water (overfilling water) coming from the cavity of the autoclave 10 through pipe 47, as will be explained later. The warm water tank 40 (and/or sometimes the hot water tank 50, if it needs to be replenished) also receives some suitable water through pipe 45 (at a temperature of between 10-20°C, such as between 12-17°C) to compensate for some water losses (for example, about 20%) happening in every sterilization cycle due to several reasons, such as water evaporation within the cavity of the autoclave 10 and water blowdown (drain).

The recovery system 25 forms a circuit in which water circulates through the warm water tank 40, hot water tank 50 and second heat exchanger 30 while the overfilling water disposed within the cavity of the autoclave 10, after a sterilization cycle, is hot enough to enable heat transfer to the water stored in tank 40, as follows. During the cooling process (in which hot overfilling water stored in the cavity of the autoclave 10 exchanges heat at heat exchanger 20 with the fresh water circulating within pipe circuitry 80), water stored in the warm water tank 40 circulates through pipe 91 towards the heat exchanger 30, where heat accumulated in the water circulating through pipe circuitry 80 is transferred to the water coming into the heat exchanger 30 through pipe 91. This warmed-up water leaves the heat exchanger 30 through pipe 92 to reach the hot water tank 50.

Preferably, warm water from the heat exchanger 30 is only pumped (by pump 98) to pipe 92 when it is possible to efficiently heat the water coming to the heat exchanger 30 from pipe 91, as explained next. It is possible to efficiently heat the water coming from pipe 91 when the temperature of the water reaching the second heat exchanger 30 through pipe 81 is a temperature higher than the temperature of the water reaching the second heat exchanger 30 through pipe 91. Preferably, warm water from the heat exchanger 30 is pumped to pipe 92 when the temperature of the water reaching the second heat exchanger 30 through pipe 81 is at least 5 degrees higher, preferably at least 10 degrees higher, more preferably at least 15 degrees higher, than the temperature of the water reaching the second heat exchanger 30 through pipe 91.

Preferably, when it is not possible to efficiently heat the water coming to the heat exchanger 30 from pipe 91, pump 98 is stopped and there is therefore no water circulation from the first tank 40 to the second heat exchanger 30 and from this one to the second water tank 50. Energy for operating the pump 98 is thus saved. Depending on the amount of water stored in tanks 40 and 50, water may flow from tank 40 to tank 50 through pipe 93.

How to determine when cooled water leaving the cavity of the autoclave 10 and stored in tank 40 can be efficiently heated with heated water circulating through pipe circuitry 80 may be done in different ways, which can be combined.

This may be done using a temperature sensing means 63, such as a temperature gauge, installed at the output of the heat exchanger 30 at pipe 92, to monitor the temperature of water leaving the heat exchanger 30 through pipe 92. Preferably, temperature is monitored continuously and is periodically sent to a controller, in such a way that a new temperature measurement is received at the controller more than 2 times per minute, such as more than 5 times per minute or more than 10 times per minute, for example every 5 seconds, every second or every half a second. When the monitored temperature is above a certain threshold (for example above about 45°C), this means that energy (i.e. heat from the water travelling through pipe circuitry 80) can be recovered. When the monitored temperature is below the threshold, it is determined that there is no point in trying to recover energy from the water travelling through pipe circuitry 80 because there is not enough heat transfer. Optionally, a second temperature sensing means 64, such as a temperature gauge, can be installed at the input of the heat exchanger 30 at pipe 91, to obtain a difference between a temperature measured at the first temperature sensing means 63 and a temperature measured at the second temperature sensing means 64, in such a way that the difference determines whether or not it is worth keeping on trying to recover energy at the heat exchanger 30. When the monitored temperature is above the threshold, warm water stored in the warm water tank 40 is pumped through pipe 91 to be stored in hot water tank 50, as already explained.

Alternatively, or in combination with the former way of determining the water temperature with temperature sensing means 63, 64, a temperature sensing means 61, such as a temperature gauge, can be disposed at pipe 81 to measure the temperature of the water leaving the first heat exchanger 20 towards the second heat exchanger 30.

Alternatively, or in combination with one or more of the temperature sensing means 63, 64, 61, when cooled water leaving the cavity of the autoclave 10 and stored in tank 40 can be efficiently heated with heated water circulating through pipe circuitry 80 can be determined through a connection (for example through modbus) with the installation controller (for example, a PLC of an autoclave 10), and enquiring when a sterilization cycle has ended, because the end of the sterilization cycle triggers the starting of the cooling cycle for the overfilling water within the cavity of the autoclave 10, and therefore the starting of the heating of the water circulating through pipe circuitry 80.

Once it has been detected, by any of the former ways, that heat can be efficiently transferred, the pump 98 can be started so that the recovery system 25 starts working.

In an example of the invention, it has been observed that this (that the monitored temperature is above the threshold), usually happens from the beginning of the cooling process (i.e. first minute) and for several minutes (such as up to 15 minutes, preferably up to 12 minutes, more preferably up to 10 minutes). The cooling process typically starts when it is determined that the sterilization cycle has ended (the sterilization cycle is normally programmed to last a period of time required to sterilize the products). During this period of time, the temperature at the output of the heat exchanger 30 (pipe 92) was observed to vary between about 65°C (at the first minute of the cooling process) and about 43°C (at the 12^{th} minute of the cooling process), which implies a thermal gap of between 48 and 26°C considering the standard temperature (about 17°C) of cooling water coming from a conventional cooling tower. During this period of time, the temperature of water circulating in pipe circuitry 80 was observed to vary between about 60 and about 80°C. The temperature of water accumulated in the hot water tank 50 was observed to vary between about 65°C (highest temperature, when water in circuitry 80 reached about between 80-90°C) and about 43°C (lowest temperature, when water in circuitry 80 got down to about 50°C). Therefore, in this example, water stored in the warm water tank 50 reached an average temperature of about 50-58°C, such as about 53-55°C. Therefore, the difference between the temperature of the water (between 50-58°C) used to replenish through pipe 15 the cavity of the autoclave 10 and the temperature of the water (about 17°C) used to replenish the cavity of the autoclave 10 in conventional retort systems not using the described recovery system 25 is of about 33-41°C, which represents the energy saving obtained with the proposed method and system.

The cooling process ends when the monitored temperature is below the threshold as detected by the temperature sensing means. At the end of the cooling process, the cavity of the autoclave 10 still has some remaining water, which is cooled water because it has travelled through pipe circuitry 70 connecting the cavity of the autoclave 10 and the heat exchanger 20. The end of the cooling process, which implies that the water contained in the cavity of the autoclave 10 is cold enough (for example, at a temperature below 45°C, such as between 40-45°C or even lower, such as between 20-38°C) to be taken out of the autoclave, triggers the emptying of the cavity of the autoclave 10. The cavity is emptied through the outlet connected to outlet pipe 47. The energy (or part thereof) obtained in this cooling process (energy transferred from the hot water retained in the autoclave 10 to the cold water circulating within pipe circuitry 80) is stored in the hot water tank 50 to be later used in subsequent sterilization cycles.

Cooled-down water extracted from the autoclave 10 is stored in the warm water tank 40. Thus, water can be reused in subsequent sterilization cycles. In the event certain amount of water is wasted, for example is evaporated in the sterilization cycle or is drained when required, some suitable water (i.e. osmosis water) may need to be provided to the warm water tank 40 through pipe 45.

Prior to starting a new sterilization cycle within the autoclave 10, and once the products (such as containers, i.e. cans, or packages of containers) to be sterilized in the new cycle are disposed within the cavity of the autoclave 10, hot water stored in the hot water tank 50 is pumped to the cavity of the autoclave 10 through pipe 15. For example, the temperature of this water may vary between about 45-58 °C, such as between 50-58°C. When the cavity is filled with water to a required level, the cavity of the autoclave 10 is hermetically sealed and a new sterilization cycle starts.

Optionally, a system for treating the water stored in the warm water tank 40 is implemented. Water stored in this tank 40, which may be water coming from the cavity of the autoclave 10 (through pipe 47), water provided for the first time (through pipe 45) and water already circulated through the recovery system 25 can be extracted through a pipe 42 from the tank 40 and taken to a water treatment system 43, where it is processed. Once treated, this water is taken back to the tank 40 through pipe 41. At the water treatment system 43 water is processed by applying any suitable water treatment process (chemical one or based on other technologies). Once treated, water is conducted back to the tank 40 by pump 48.

The water treatment system 43 is not only used during the cooling cycle (when water is pumped by pump 98 through the recovery system 25), but also when water is stored in the tanks 40, 50 when the autoclave 10 is not in operation (for example at night or during the weekends). In these circumstances, the temperature of the water stored in the tanks 40, 50 may fall under 50°C, in which case the pump 98 is switched on so that water recirculates through the recovery system 25 and is taken to the water treatment system 43.

The recovery system 25 also has a bypass pipe 97 disposed between pipes 92 and 91 in pipe circuitry 90 to be used in the event that water stored in tanks 40, 50 needs to be recirculated, for example if there is risk of microbiological contamination, but it is not possible to exchange heat, and therefore water needs to circulate but not through heat exchanger 30. In these situations, water circulating from tank 40 to pipe 91 is bypassed through bypass 97 to tank 50 and back to tank 40 through pipe 93. Deterioration or damage of the heat exchanger 30 and/or of its efficiency is thus prevented.

The retort system 100 may have a plurality of batch autoclaves. All or some of the autoclaves of the plurality of autoclaves may share a same recovery system 25. For example, the water extracted from several autoclaves 10 may be delivered to a common warm water tank 40. Also, the water used to replenish the cavities of several autoclaves 10 may be taken from a common hot water tank 50. For example, a single heat exchanger 30 can be shared by a plurality of autoclaves. All the autoclaves are connected to a control system which detects which autoclave is starting a cooling cycle. The heat exchanger 30 is then switched to work with the identified autoclave.

In this text, the term "comprises" and its derivations -such as "comprising", etc.- should not be understood in an excluding sense, that is, these terms should not be interpreted as excluding the possibility that what is described and defined may include further elements, steps, etc.

The invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. A retort system (100) comprising:
at least one autoclave (10) for sterilizing a product contained within a cavity of the at least one autoclave (10), wherein in use of the retort system (100), the cavity of the at least one autoclave (10) is partially filled with water, the product being sterilized by transferring heat from said water;
a first heat exchanger (20) configured to cool down the water remaining within the cavity of the at least one autoclave (10) after a sterilization cycle has been completed, the first heat exchanger (20) cooling down said water by transferring heat of said water to cooling water;
a recovery system (25) comprising:
a first tank (40) configured to store water coming from the cavity of the at least one autoclave (10) after a sterilization cycle has been completed and the water remaining within the cavity after the sterilization cycle has been cooled down;
a second heat exchanger (30) configured to transfer the heat previously transferred to the cooling water at the first heat exchanger (20) to the water stored in the first tank (40); and
a second tank (50) configured to store the water heated at the second heat exchanger.

2. The retort system of claim 1, further comprising:
a first piping system (70) disposed between the at least one autoclave (10) and the first heat exchanger (20), the first piping system (70) for taking water from the at least one autoclave (10) to the heat exchanger (20) and for returning cooled-down water from the heat exchanger (20) to the autoclave (10);
a second piping system (80) disposed between the first heat exchanger (20) and the second heat exchanger (30), the second piping system (80) for circulating cooling water between the first heat exchanger (20) and the second heat exchanger (30); and
a third piping system (91, 92, 93) for circulating the water stored in the first tank (40) through the second heat exchanger (30) so that said water is heated with the heat transferred from the water circulating through the second piping system (80).

3. The retort system of either claim 1 or 2, wherein the first tank (40) is further configured to receive and store treated water different from the water coming from the cavity of the at least one autoclave (10).

4. The retort system (100) of any one of the preceding claims, further comprising a pipe (15) for taking water stored in the second tank (50) to the at least one autoclave (10) prior to starting a sterilization cycle.

5. The retort system (100) of any one of the preceding claims, further comprising means for pumping water stored in the first water tank (40) to the second water tank (50).

6. The retort system (100) of any one of the preceding claims, further comprising at least one temperature sensing means to measure the temperature at at least one point in the retort system (100).

7. The retort system (100) of any one of the preceding claims, further comprising a system (43) for treating the water stored in the first water tank (40).

8. The retort system (100) of any one of the preceding claims, wherein the cooling water comes from a cooling tower.

9. The retort system (100) of any one of the preceding claims, wherein the product to be sterilized is kept within one or more containers disposed within said cavity.

10. The retort system (100) of any one of the preceding claims, wherein the product to be sterilized is an edible product.

11. The retort system (100) of any one of the preceding claims, wherein the at least one autoclave (10) is configured to sterilize a product by applying a temperature and pressure treatment for a certain time.

12. A method for water and/or energy recovery in an installation (100) comprising at least one autoclave (10) for sterilizing a product contained with a cavity of the at least one autoclave (10), the method comprising:
disposing a product to be sterilized within the cavity of the at least one autoclave (10);
partially filling the cavity of the at least one autoclave (10) with water;
sterilizing the product by applying a temperature and pressure treatment for a certain time;
after the product is sterilized, at a first heat exchanger (20) cooling down the water comprised in the cavity of the at least one autoclave (10) using cooling water while heat is transferred from the water comprised in the cavity to the cooling water; and
at a second heat exchanger (30), using the heated cooling water to warm up water to be provided to a cavity of at least one autoclave (10) prior to starting a subsequent sterilization cycle.

13. The method of claim 12, further comprising storing water coming from the cavity of the at least one autoclave (10) after a sterilization cycle has been completed and the remaining water within the cavity has been cooled down in a first tank (40).

14. The method of either claim 12 or 13, further comprising storing the water warmed up at the second heat exchanger (30) in a second tank (50).
